# EUROPEAN PATENT APPLICATION

(11) **EP 3 753 558 A1**
(43) Date of publication of application: **23.12.2020**
(21) Application number: 19181742.8
(22) Date of filing: 21.06.2019
(51) Int. Cl.: A61K 31/198, A61K 45/06, A61P 27/16

(54) **THYROID HORMONE FOR USE IN THE TREATMENT OF A PERIPHERAL VESTIBULOPATHY**

(71) Applicant: CENTRE NATIONAL DE LA RECHERCHE SCIENTIFIQUE -CNRS-, 75016 Paris (FR); Universite d'Aix-Marseille (AMU), 13284 Marseille Cedex 07 (FR)
(72) Inventor: TIGHILET, Brahim, 13015 Marseille (FR); CHABBERT, Christian, 30000 Nimes (FR); RASTOLDO, Guillaume, 06310 Beaulieu sur Mer (FR); PERICAT, David, 13850 Greasque (FR)
(74) Representative: Macquet, Christophe

(57) **Abstract**

The invention relates to a thyroid hormone for use in the treatment of a peripheral vestibulopathy in a patient in need thereof, the patient being not diagnosed with hypothyroidism.

## Description

### TECHNICAL FIELD

The invention relates to a thyroid hormone, and to pharmaceutical compositions comprising such a thyroid hormone, for use in the treatment of a peripheral vestibulopathy in a patient in need thereof.

### BACKGROUND OF THE INVENTION

Vestibular loss causes vestibular syndrome in most species including human. This syndrome is characterized by a cascade of functional disorders including postural imbalance, impaired walking, spontaneous nystagmus, impaired vestibulo ocular reflexes, as well as cognitive and neurovegetative disorders. In human, as in animal models of vestibular disorders of a peripheral origin, i.e. peripheral vestibulopathies, the vestibular syndrome is generally composed of several phases, the amplitude of which depends on the type, stage and severity of the peripheral damage. The "acute" phase characterizes the period in which static disorders (posturo-locomotor symptoms and spontaneous nystagmus at rest) are the most prominent. This phase generally lasts several hours, but may extend to days. Subsequently, the various symptoms that constitute the vestibular syndrome progressively decline, each with its own kinetics, generally leading to a rapid and complete disappearance of static deficits and an often slower and incomplete regression of dynamic deficits. This phenomenon is called "vestibular compensation" (Smith and Curthoys, 1989, Lacour and Tighilet, 2010, Lacour et al., 2016). Two major and interdependent neurobiological phenomena occurring in the deafferented vestibular environment are closely related to the restoration of vestibular functions: i) the restoration of a level of homeostatic excitability which restores the electrophysiological balance between the two homologous vestibular nuclei(VN)(Smith and curthoys 1989; Ris et al., 1995, Curthoys, 2000; Darlington et al., 2002,) and ii) the expression of neurogenesis (Tighilet et al., 2007, Dutheil et al., 2009, Tighilet and Chabbert, 2019).

The priority of the deafferented vestibular environment is to promote the expression of these two mechanisms essential for vestibular functional recovery. Different strategies are put in place to restore the level of excitability. The inventors have demonstrated that the deafferented vestibular environment is reconfigured by reducing the expression of cotransporteurs KCC2, which results in a depolarizing action of GABA during the first three days after unilateral vestibular neurectomy (UVN) (Dutheil et al., 2016). Other modulators of the neuronal excitability such as the SK (small conductance calcium-activated potassium) channels increase their level of expression in the VN after vestibular loss (Tighilet et al., 2019).

The pharmacological modulation of these specific central reactive mechanisms modifies the kinetics of functional restoration. Indeed, the blockage of neurogenesis by an antimitotic agent (Dutheil et al., 2009) or its potentiation by a neurogenic factor such as BDNF (Dutheil et al., 2016) significantly modifies the restoration of postural and locomotor balance. Similarly, the pharmacological blocking of SK channels by apamine, in vestibulo-injured feline facilitates the restoration of the posturo-locomotor function and gaze stabilization (Tighilet et al., 2019). This effect has been postulated to result from the inhibition of the hyperpolarization phase of the action potential in neurons that express this channel type.

However, and based on the above, there remains a need for discovering active ingredients and pharmaceutical compositions that alleviate the vestibular syndrome and facilitate the vestibular compensation in cases of a peripheral vestibulopathy.

### SUMMARY OF THE INVENTION

In accordance with a first aspect, the invention relates to a thyroid hormone for use in the treatment of a peripheral vestibulopathy in a patient in need thereof, the patient not being diagnosed with hypothyroidism.

The inventors observe that after a peripheral vestibular insult in the rodent, a treatment with thyroid hormone allows a significant reduction of the induced vestibular deficits, in particular a decrease of the vestibular deficit signs and general behavioral alterations during the acute phase, accompanied by improvement of walking and speed motion over several days.

Preferentially, - the patient is not being diagnosed with hyperthyroidism; - the vestibulopathy is a lesional peripheral vestibulopathy; - the vestibulopathy is selected from the group consisting of peripheral vestibular neuritis, labyrinthitis or Ménière's disease; - the thyroid hormone is triiodothyronine or thyroxine; - the thyroid hormone is thyroxine; - the thyroid hormone is administered in an amount superior or equal to 5 µg/kg, advantageously in an amount ranging from 5 µg/kg to 100 pg/kg; - the thyroid hormone is administered at a level of approximately 10 µg/kg; and - the thyroid hormone is administered once a day during the five days following the insult, preferentially during the three days following the insult.

In accordance with a second aspect, the invention relates to a pharmaceutical composition comprising at least a thyroid hormone according to the invention, and at least one pharmaceutically acceptable excipient, for use in the treatment of a peripheral vestibulopathy in a patient in need thereof, the patient not being diagnosed with hypothyroidism.

Preferentially, - the composition is administered by intraperitoneal route.

### BRIEF DESCRIPTION OF THE FIGURES

Other features and aspects of the present invention will be apparent from the following description and the accompanying drawings, in which:
- Figure 1 illustrates the experimental protocol elaborated for studying the effects of unilateral vestibular neurectomy and the consequences of thyroxin (T4) intraperitoneal injection, on the time course of vestibular and posturo-locomotor function recovery (n=8 animals per group);
- Figure 2 illustrates the weight applied to the left paws (front and rear) in pre-operative situation and at D30 after the lesion;
- Figure 3 illustrates the acceleration of the support surface recovery time-course, upon T4 treatment after unilateral vestibular neurectomy, and the curves illustrate the mean postoperative recovery of the support surface in the two experimental groups of rats (UVN-NaCl in black and UVN-T4 in grey);
- Figure 4 illustrates the effect of T4 on the evolution of animal's locomotor velocity after unilateral vestibular neurectomy;
- Figure 5 illustrates the effect of T4 on the evolution of animal's distance moved after unilateral vestibular neurectomy;
- Figure 6 illustrates the effect of T4 on the immobility time of the animals; and
- Figure 7 illustrates the acceleration of vestibular syndrome recovery time-course based on the vestibular score, upon T4 treatment after unilateral vestibular neurectomy, and the curves illustrate the mean postoperative recovery of the vestibular score in the two experimental groups of rats (UVN-NaCl in black and UVN-T4 in grey).

### DETAILED DESCRIPTION OF THE INVENTION

The invention provides a thyroid hormone for use in the treatment of a peripheral vestibulopathy in patients in need thereof, the patients not being diagnosed with hypothyroidism.

According to the present invention, the expression "between... and..." used to define ranges of values should be understood as integrating the lower and upper limits of these ranges.

A "thyroid hormone", as referred herein, can be synthetic or natural. In nature, thyroid hormones are produced and released by the thyroid gland. They are tyrosine-based hormones that are primarily responsible for regulation of metabolism. According to the invention, the terms "thyroid hormones" refer to triiodothyronine, thyroxine, and their isomers, metabolites or derivatives. For example, the thyroid hormone for use according to the invention can be chosen in the group consisting of 3,5,3' triiodothyronine (T3), 3,3',5'-triiodothyronine (reverse T3), 3,3'-diiodothyronine (T2), 3'-monoiodothyronine (T1), thyronine (T0), or 3,5,3',5'-tetraiodo-L-thyronine (T4). Preferentially, the thyroid hormone for use according to the invention is 3,5,3',5'-tetraiodo-L-thyronine, also named thyroxine. In the following, the terms "thyroxine", "3,5,3',5'-tetraiodo-L-thyronine" or "T4" are used indifferently.

As used herein, the terms "treating", "treatment", and "therapy" refer to a symptomatic or curative therapy. Accordingly, the aim of the invention is to provide a relief of the peripheral vestibulopathy symptoms or an improvement of the patient's condition by alleviating the symptoms (nystagmus, postural imbalance, erroneous sensation of movement, dizziness) and promoting the vestibular functional recovery (gaze stabilization, static and dynamic balance).

The terms "vestibulopathy", "vestibular disorder" or "vestibular syndrome" refer to a disorder of the vestibular system which includes the parts of the inner ear and brain that process the sensory information involved with controlling balance and gaze stabilization. If an injury, or a disease, damages these areas, vestibular disorders appear.

The terms "peripheral vestibulopathies", i.e. peripheral vestibular disorder, refers to a dysfunction of the balance sensory organs of the inner ear, as opposed to central vestibular disorders referring to a dysfunction of one or more parts of the central nervous system involved in processing balance and spatial information. The peripheral vestibular disorders are caused by damages and/or dysfunctions in the semicircular channels, the vestibule (utricle and saccule), or the vestibular nerve. As an example, the main causes of peripheral vestibular syndrome are Benign Positional Paroxysmal Vertigo (BPPV), Ménière's disease, vestibular neuritis and labyrinthitis. For example, vestibular disorders are selected from the group consisting of vertigo/dizziness, nystagmus, balance unsteadiness, loss of muscular tonus, often accompanied by neurovegetative manifestations such as nausea, vomiting, and salivation, and perceptive-cognitive manifestations such as alteration of the body schema, subjective vertical perception, spatial disorientation or sleep.

The terms "lesional peripheral vestibulopathies", "lesional peripheral vestibular disorder", "lesional peripheral vestibular deficit" or "lesional peripheral vestibular syndrome" refers to peripheral vestibular disorders wherein lesions on inner ear cells, the vestibular nerve and/or the contact between them are present or will appear during the disorder time course. In this case, the functionality of the vestibule is impaired. Lesional peripheral vestibular disorders include : - vestibular disorders wherein an infection inflames the inner ear and or the vestibular nerve, inducing reversible and/or irreversible damages, one example of conditions from this group is vestibular neuritis; - vestibular disorders wherein inner ear fluid levels are affected (abnormalities in the quantity, composition, and/or pressure of the endolymph), these disorders usually develop lesions during the disease time course, such as Menière's disease and secondary endolymphatic hydrops;-vestibular disorders induced by insults or lesions of the vestibular end-organs, such as vertigo causes by local ischemia, excitotoxicity. Examples of lesional vestibular disorders that are contemplated by the invention include but are not limited to neuritis, vestibular neuritis, labyrinthitis, viral endolymphatic labyrinthitis, druginduced ototoxicity, Menière's disease, endolymphatic hydrops, head trauma with lesional vestibular deficits, labyrinthine haemorrhage, chronic or acute labyrinthine infection, serious labyrinthine, barotraumatism, autoimmune inner ear disease, presbyvestibulia, toxic vestibular impairments.

According to the invention, the peripheral vestibulopathy is preferentially a lesional peripheral vestibular disorder such as Ménière's disease, vestibular neuritis or labyrinthitis.

The patients are mammals and, more particularly, humans. The patients considered in the present invention are not diagnosed with hypothyroidism. Because they are not diagnosed with hypothyroidism, they are not treated for this disorder. Advantageously, the patients are not suffering from hypothyroidism, the hypothyroidism being diagnosed or not. Thus, they are not hypothyroid patients i.e. they are not patients suffering from hypothyroidism. Therefore, the considered patients may be "thyroid healthy patients" or patients being diagnosed with hyperthyroidism and/or patients suffering from hyperthyroidism. Preferentially, the patients of the invention are not being diagnosed with hyperthyroidism either, or the patients are not suffering from hyperthyroidism, the hyperthyroidism being diagnosed or not. In other words, the patients of the invention have not been diagnosed with, or are not suffering from, hypothyroidism or hyperthyroidism, and are therefore considered as "thyroid healthy patients". Patients being diagnosed with hypo- or hyperthyroidism usually undergo a treatment based on thyroid hormones to treat their thyroid disorder. This group of patients is not encompassed within the scope of the present invention.

According to the invention, the thyroid hormone for use is administered once a day, during the five days following the insult. Preferentially, the thyroid hormone is administered once a day, during the three days following the insult.

According to the invention, the thyroid hormone for use is administered in an amount superior or equal to 5 µg/kg of body weight. Advantageously, the thyroid hormone for use is administered in an amount ranging from 5 µg/kg to 100 µg/kg of body weight, more advantageously in an amount ranging from 5 µg/kg to 50 µg/kg, and even more advantageously in an amount ranging from 8 µg/kg to 20 µg/kg. Preferentially, the thyroid hormone for use is administered at a level of approximately 10 µg/kg of body weight.

In another aspect, the invention relates to a pharmaceutical composition for use in the treatment of a peripheral vestibulopathy in a patient in need thereof, the patient not being diagnosed with hypothyroidism. The pharmaceutical composition for use according to the invention comprises at least a thyroid hormone as described above, and at least one pharmaceutically acceptable excipient.

The pharmaceutical composition is adapted for use in the treatment of a peripheral vestibulopathy. The peripheral vestibulopathies are caused by dysfunction in the semicircular channels, the vestibule (utricle and saccule), or the vestibular nerve. As an example, the main causes of peripheral vestibular syndrome are Benign Positional Paroxysmal Vertigo (BPPV), Meniere's disease, vestibular neuritis and labyrinthitis. For example, peripheral vestibular disorders are selected from the group consisting of vertigo/dizziness, nystagmus, balance unsteadiness, loss of muscular tonus, often accompanied by neurovegetative manifestations such as nausea, vomiting, and salivation, and perceptive-cognitive manifestations such as alteration of the body schema, subjective vertical perception, and spatial disorientation.

According to the invention, the patient in need thereof has not been diagnosed with hypothyroidism and/or is not suffering from hypothyroidism. Preferentially, the patient of the invention has not either been diagnosed with hyperthyroidism and/or is not suffering from hyperthyroidism. In other words, the patient of the invention has not been diagnosed with, and/or is not suffering from, hypothyroidism or hyperthyroidism.

According to an embodiment of the invention, the pharmaceutical composition can be administered per os, by intraperitoneal or by intravenous route. Preferentially the pharmaceutical composition for use according to the invention is administered by intraperitoneal route.

In the present invention, the thyroid hormone comprised in the pharmaceutical composition for use is administered once a day, during the five days following the insult. Preferentially, the thyroid hormone is administered once a day, during the three days following the insult.

According to the invention, the thyroid hormone comprised in the pharmaceutical composition for use is administered in an amount superior or equal to 5 µg/kg. Advantageously, the thyroid hormone is administered in an amount ranging from 5 µg/kg to 100 µg/kg, more advantageously in an amount ranging from 5 µg/kg to 50 µg/kg, and even more advantageously in an amount ranging from 8 µg/kg to 20 µg/kg. Preferentially, the thyroid hormone comprised in the pharmaceutical composition for use is administered at a level of approximately 10 µg/kg.

According to a specific embodiment of the invention, the thyroid hormone 3,5,3',5'-tetraiodo-L-thyronine comprised in the pharmaceutical composition for use, is administered intraperitoneally, once a day, during the three days following the insult, at a level of approximately 10 µg/kg.

### METHODS AND EXAMPLES

### METHODS

The following methods were used for the implementation of the invention, and in the examples:

### Unilateral Vestibular Neurectomy

Animals were anesthetized using isoflurane gas (compact anesthesia module + ventilator - Minerve). For induction: 5% isoflurane at an air flow rate of 1,5 liters per minute. For Maintenance: 3 to 3,5% isoflurane at an air flow rate of 0,4 liter per minute. The animal's breathing was controlled by the ventilator from the isoflurane station while animal's temperature was maintained by a heated blanket with a double temperature probe: a rectal probe and a probe incorporated with a heating blanket in order to maintain an optimal temperature of the animal during surgery. A subcutaneous injection of Buprenorphine (Buprecare 0,05 mg/kg) was performed 30min before starting surgery. Then, animals were submitted to a left-side vestibular nerve section. Unilateral Vestibular Neurectomy (UVN) was performed under visual control through a dissecting microscope. The vestibular nerve was sectioned at a post ganglion level as close as possible to the brainstem. Before awakening the animal by intraperitoneal injection of Antisedan® (Orion-Pharma; 1 mg/kg), a solution of Ringer Lactate (Virbac; 10 ml/kg) was administered subcutaneously in order to alleviate the dehydration resulting from the inability of the animal to drink normally as a result of injury. Upon waking, all animals exhibited a vestibular syndrome composed of both a tumbling behavior and spontaneous nystagmus that confirmed the achievement of the unilateral vestibular neurectomy. The successfulness of the surgery was attested at the behavioral level by the presence of a characteristic vestibular syndrome and at the histological level, by the observation under optical microscopy of the full section of the 8th cranial nerve between Scarpa ganglion and vestibular nuclei from the brainstem (see Pericat et al., 2017 for details).

### Animals and treatment

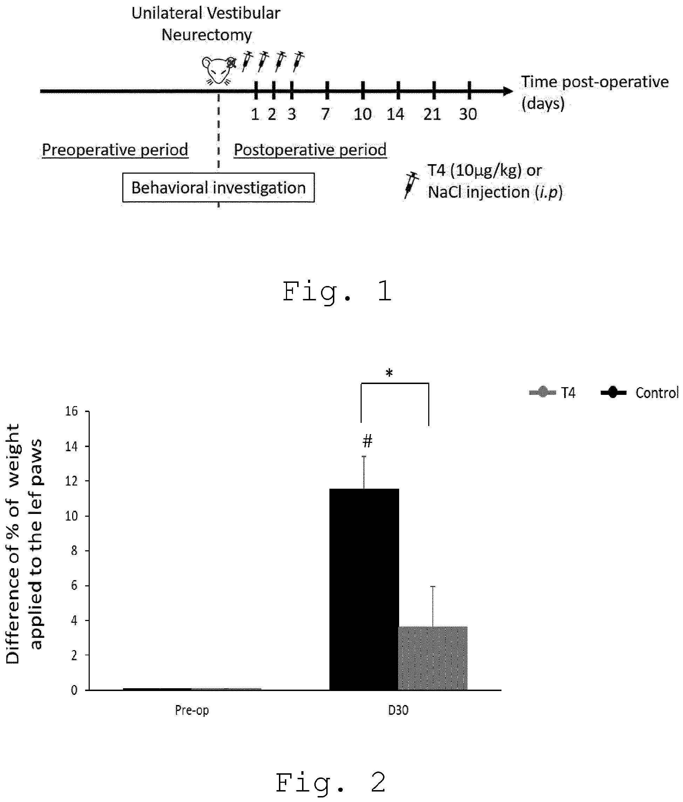

Female Long Evans rats (200-250g, age 3 months at time of UVN) were housed individually per cage after the surgery in a temperature-controlled room with 12h light/dark cycle, with free access to food and water. In total 16 animals were included in this study. 8 animals underwent left UVN and were injected (i.p) with a saline solution just after UVN and at day 1, 2 and 3 as a control group. The remaining 8 rats were subjected to left UVN and injected (i.p) with thyroxin solution (T4) at a dose of 10µg per kg just after UVN and at day 1, 2 and 3 as a T4 treatment group (Fig.1). Behavioral tests were realized 10 min after injection for both control and T4 treatment group.

Figure 1 illustrates the experimental protocol elaborated for studying the effects of UVN and the consequences of thyroxin (T4) intraperitoneal injection on the time course of vestibular and posturo-locomotor function recovery (n=8 animals per group). In the preoperative period, the vestibular function, posture function and locomotor activity are evaluated. The vestibular function is evaluated qualitatively by determining a vestibular score, the posture function is evaluated by a support surface and a weight distribution quantitative analysis, and the locomotor activity is evaluated with a video-tracking as explained hereinafter. In the postoperative period, the vestibular function recovery, posture function recovery and locomotor activity recovery are investigated.

### Criteria of Exclusion

1 rat of the T4 treatment group was excluded from the study because of abnormalities in behavioral scoring.

### Posturo-locomotor syndrome quantification (DWB® from BIOSEB)

In order to quantify the posturo-locomotor syndrome following the UVN, the inventors used a device (DWB® from BIOSEB) measuring the weight distribution at all contact points of the animal body with the ground. This device consists of a Plexiglas cage (25x25 cm) in which the animal can move freely. The floor of this cage is fully covered with a 2000 force sensors plate. Sensors detect vertical pressure at a frequency of 30 Hertz. The sensors are connected to an electronic interface that converts the current flowing through it into a measure of weight, the whole being connected to a computer. The cage is closed by a lid on which is attached a high definition camera, also connected to the computer through a USB cable.

Analysis of the various parameters was carried out at different pre- and post-operative times: a first acquisition was made the day before the lesion, serving as a reference value, and then acquisitions were performed at days (D) 1, 2, 3, 7, 10, 17, 21 and 30 post-lesion. For each acquisition session, the animal was placed in the device and moved freely during the 5 minutes recording period.

The analysis allowed acquiring many parameters. The inventors were interested in the following: the weight distributed on the left paws (front and rear), which are the paws ipsilateral of the unilateral vestibular lesion. This data reflects an asymmetrical weight distribution of the rat on the lateral axis. In order to compensate for the weight variability of the animals and the variability of the time analyzed over the 5 min acquisition, each of the parameters studied was expressed as a percentage of the animal weight on the day of acquisition and as a percentage of the analyzed time. Sequences in which the software cannot clearly monitor the paws weight and position were omitted, as well as situations in which the rats put their paw against the wall.

### Support Surface

Static postural deficits and recovery were evaluated by measuring the support surface delimited by the four legs of the rat after the tail hanging landing test. This test consisted in taking the animal by the tail and lifting it vertically over a height of about 50 cm (lift duration 2 s; position holding at upper position: 1 s). The support surface can be regarded as a good estimate of postural control because it reflects the rat's behavioral adaptation compensating the static vestibulospinal deficits induced by the vestibular lesion. To quantify the support surface, rats were placed in a device with graduated transparent floor that allowed the animals to be filmed from underneath. A scale drawn on the bottom served to take measurements of the four paws location. When the animal landed after the tail hanging landing test and touched the ground, the inventors took a capture of the four paws location. Twenty repeated measurements were done for each rat tested at each postoperative time, and an average was calculated for each experimental session. A first acquisition was made the day before the lesion, serving as a reference value, and then acquisitions were performed at days (D) 1, 2, 3, 7, 10, 17, 21 and 30 post-lesion. The support surface was measured using an image analysis system developed by inventors' team on Matlab. An average was calculated for each post-lesion time. Data recorded after unilateral vestibular lesion were compared to the mean pre-lesion values using individual references, which means each animal acted as its own control.

### Video-Tracking

Animals were placed in an open field (80×80×40 cm). Animals were placed individually in the center of the platform and their behavior was recorded for 10 min using a digital camera for later analysis with EthoVision™ XT 14 software (Noldus). The surfaces of the open-field were cleaned thoroughly between animals. To minimize stress, the room was lit as dimly as possible while allowing the inventors to clearly discern the rats. At the beginning of the session, the rats were placed individually on the right side of the field, with head in front of the wall. Detection thresholds were set with the brighter and darker scale advanced model-based detection (range 17 to 97 in bright and 32 to 255 in dark, 25 fps). Ethovision™ device automatically detected the nose, middle point, and tail base of each animal throughout recordings. Three variables were selected for analysis the distance moved (cm), the mean locomotor velocity (cm/s) and the Time of immobility (s) of animals. "Distance moved" and "Mean locomotor velocity" were detected from the center point. "Not Moving" were detected from the center point with thresholds of 2 and 1.75 cm/s. For all parameters, the inventors used the minimal distance moved smoothing method to filter out small movements (< 0,7cm) of the subject's center point that are caused by random noise rather than the subject's spatial displacement. A first acquisition was made the day before the lesion, serving as a reference value, and then acquisitions were performed at days (D) 1, 2, 3, 7, 10, 17, 21 and 30 post-lesion.

### Vestibular score

In parallel with the quantitative analysis indicated above, the inventors evaluated qualitatively the vestibular syndrome following unilateral vestibular neurectomy (UVN). Behavioral symptoms of vestibular imbalance were scored for 10 components after UVN: tail hanging reflex, landing reflex, rearing, grooming, displacement, head-tilt, barrel rolling, retropulsion, circling, bobbing.
- Tail hanging reflex: Animals were lifted from the ground at the base of the tail and body rotation was scored from 0 point (no rotation) to 3 points (several rotations of 360°)
- Landing reflex: After animals were lifted from the ground at the base of the tail, the inventors scored the first 3 landings reflexes from 0 (presence of a extension of forelimb while landing) to 3 points (absence of extension of forelimb while landing)
- Rearing: the ability of the rat to rear was scored from 0 point (rearing is observed) to 1 point (rearing is absent)
- Grooming: the ability of the rat to groom correctly were scored as follows: 0 point (correctly grooming of full body) 1 point (groom of the face, belly and flanks but not the base of the tail), 2 points (grooming of the face and belly), 3 points (grooming of the face), 4 points

### (inability of the animal to groom himself)

- Displacement: quality of the displacement of the rat was scored from 0 (displacement of the rat with no visible deficit) to 3 points (several deficits in the displacement of the rat)
- Head tilt was scored by estimating the angle between the jaw plane and the horizontal plane with 0 points (absence of a head-tilt) to 3 points (for a 90° angle)
- Barrel rolling was scored as follows: 0 points (absence of barrel rolling), 1 point (barrel rolling evoked by an acceleration in the vertical axis of the rat in inventors' hand), 2 points (spontaneous barrel rolling)
- Retropulsion characterizes backwards movements and was score from 0 (absence of retropulsion) to 1 point (presence of retropulsion)
- Circling was scored from 0 point (absence of circling behavior) to 1 point (presence of circling behavior)
- Bobbing that is a cephalic nystagmus is attributed to a consequence of oscillopsia (a visual disturbance in which objects in the visual field appear to oscillate) and was scored from 0 point (absence of bobbing) to 1 point (presence of bobbing)

A first acquisition was made the day before the lesion, serving as a reference value, and then acquisitions were performed at days (D) 1, 2, 3, 7, 10, 17, 21 and 30 post-lesion.

### Statistical analysis

Using Graphpad Prism6 software, effects of lesion and of time course of thyroxin (T4) treatment (T4 group) and saline treatment (control group) on the vestibular syndrome was tested by means of two-way ANOVA, followed by adapted post-hoc tests between groups (Tukey's test) where P<0.05. All data were expressed as the mean ± standard error of the mean (S.E.M.) and a P value of <0.05 was taken as the minimum level of significance.

### EXAMPLES

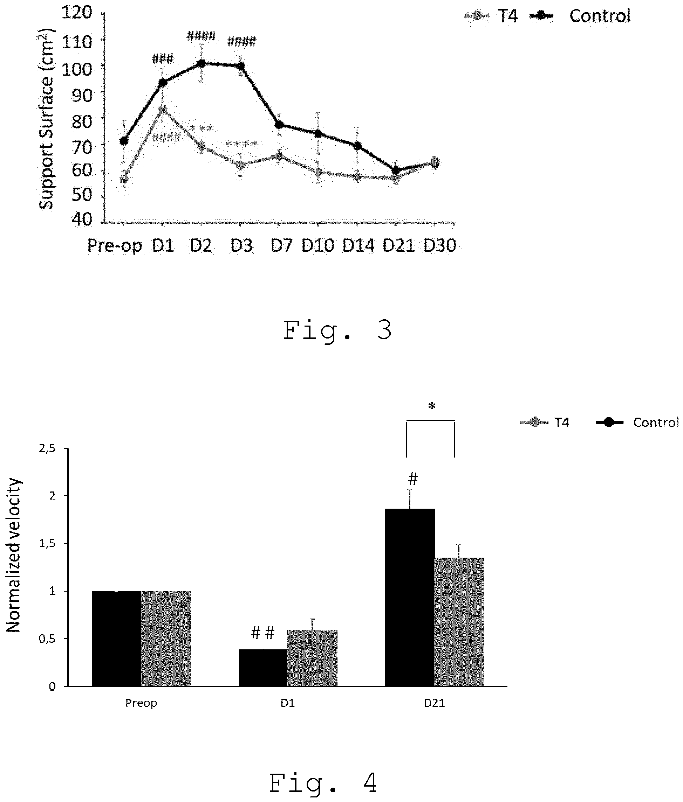

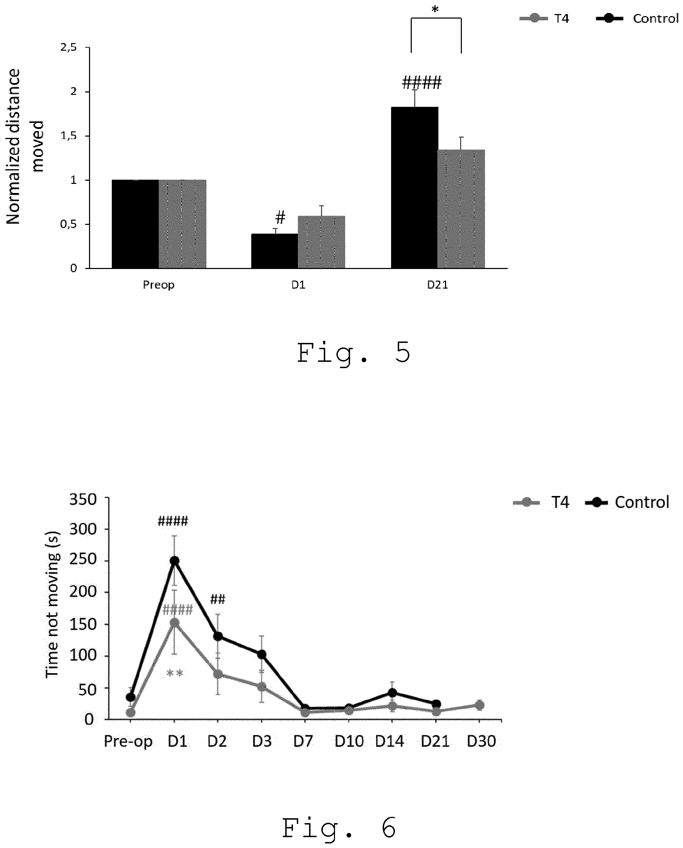

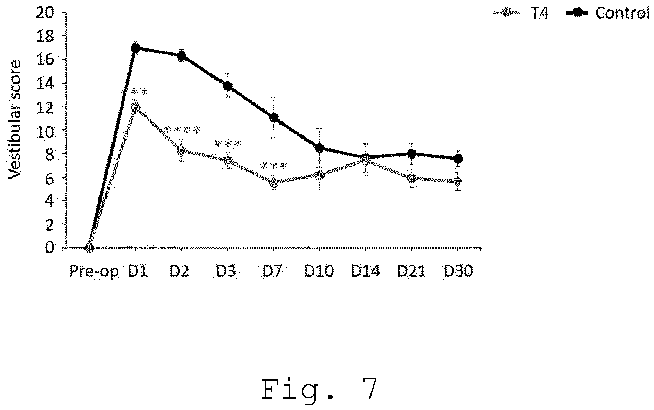

Note that in all figure below, the symbol "*" represents the difference between control and T4 treatment group while the symbol "#" represents the difference relative to the pre-operative stage.
"*" or "#" is indicative of a significant difference (p < 0,05), "**" or "##" is indicative of very significant difference (p < 0,01), "***" or "###" is indicative of highly significant difference (p < 0,001), and "****" or "####" is indicative of very highly significant difference (p < 0,0001) compared to a negative control group, two-way ANOVA.
Example 1: Effect of the thyroxine treatment on the weight applied to the left paws of the rat unilateral vestibular neurectomy model.
   Figure 2 illustrates a percentage of weight applied to the left paws (front and rear) of the animals, in pre-operative situation and at D30 after the lesion. A significant difference of the percentage of weight applied to the left paws can be observed between the control vs. the T4 treated animals at D30. While, control animals switch their weight to the left significantly on D30, the animals treated with T4 do not switch their weight to the ipsilesional side. In conclusion, T4 administration significantly eliminates the asymmetric weight distribution in vestibulo-injured animals.
Example 2: Acceleration of the support surface recovery time-course, upon T4 treatment after unilateral vestibular neurectomy
   Figure 3 illustrates the time-course recovery of the support surface after unilateral vestibular neurectomy. The curves illustrate the mean postoperative recovery of the support surface in the two experimental groups of rats (UVN-NaCl in black and UVN-T4 in grey). A significant difference can be observed between the control and the T4 treated animals, at D2 and D3. A compensation of control animals from D7 can be observed, while animals treated with T4 compensate from D2. It can therefore be concluded that T4 speeds up the recovery of animals on D2 and reduces the state of crisis observed on D2 and D3 compared to control animals.
Example 3: Effect of T4 on the evolution of animal's locomotor velocity after unilateral vestibular neurectomy
   Figure 4 illustrates the effect of T4 on the evolution of animal's locomotor velocity after unilateral vestibular neurectomy. The control animals reduce significantly their velocity at D1 (p < 0.01, ##) and then increase their velocity at D21 compare to the pre-operatory delay (p < 0.05, #). In animals treated with T4, the variation of velocity never reached a statistically significant difference relative to the pre-operatory condition. Statistically significant difference between control group and T4 treated animals is observed at D21 (p < 0.05, *). In conclusion, a T4 treatment reduces the alterations of velocity of vestibulo-injured animals.
   The normalized velocity is obtained by dividing the analyzed value by the pre-operatory (pre-op) value. Each rat will therefore have an identical pre-op value equal to 1. The velocity, at each measured time (D), are calculated with the following formula: (D value) / (Pre-op value). A normalized value greater than 1 indicates an increase over the pre-operatory conditions while a normalized value less than 1 indicates a decrease over the pre-operatory conditions.
Example 4: Effect of T4 on the evolution of animal's distance moved after unilateral vestibular neurectomy
   Figure 5 illustrates the effect of T4 on the evolution of animal's distance moved. The control animals reduce significantly their distance moved at D1(p < 0.05, #) and then increase their distance travelled at D21 compare to the pre-operatory delay (p < 0.0001, ####). In animals treated with T4, the variation of distance travelled never reached statistically significant difference relative to the pre-operatory condition. A statistically difference between control group and T4 treated animals is observed at D21 (p < 0.05, *). It can therefore be concluded that the T4 treatment reduces the altered locomotor activity in vestibulo-injured animals.
   The normalized distance moved is obtained through the same method as explained in Example 3, with the formula: (D value) / (Pre-op value), the D value being the rat's distance moved at each measured time.
Example 5: Effect of T4 on the immobility time of the animals
   Figure 6 illustrates the effect of T4 on the immobility time of the animals. A significant difference between the control group and the T4 treated group at D1 can be observed. The control animals spend significantly more time not moving ("freezing" behaviour) at D1 and D2 compared with treated animals that spend more time not moving only at D1. In conclusion, the T4 treatment significantly reduces the freezing behaviour of vestibulo-injured animals at D1.
Example 6: Acceleration of vestibular syndrome recovery time-course based on the vestibular score, upon T4 treatment after unilateral vestibular neurectomy
   Figure 7 illustrates the vestibular syndrome recovery time-course based on the vestibular score, after unilateral vestibular neurectomy. The curves illustrate the mean postoperative recovery of the vestibular score in the two experimental groups of rats (UVN-NaCl in black and UVN-T4 in grey). A significant difference between the control group and the T4 treated group can be observed at: D1, D2, D3 and D7. The control animals compensate at D10 while animals treated with T4 compensate at D2.

Therefore, it can be concluded that the T4 treatment significantly accelerates the vestibular function recovery of animals and reduces the acute vestibular syndrome expressed the first post lesion week (D1-D7).

### REFERENCES

Curthoys IS (2000) Vestibular compensation and substitution. Current Opin Neurol 13(1):27-30.
Darlington CL, Dutia MB, Smith PF. The contribution of the intrinsic excitability of vestibular nucleus neurons to recovery from vestibular damage. Eur J Neurosci (2002) 15(11): 1719-27.
Dutheil S, Watabe I, Sadlaoud K, Tonetto A, Tighilet B (2016) BDNF Signaling Promotes Vestibular Compensation by Increasing Neurogenesis and Remodeling the Expression of Potassium-Chloride Cotransporter KCC2 and GABA A Receptor in the Vestibular Nuclei. J Neurosci 36(23):6199-6212. doi:10.1523/JNEUROSCI.0945-16.2016
Dutheil S, Brezun JM, Leonard J, Lacour M, Tighilet B (2009) Neurogenesis and astrogenesis contribute to vestibular compensation in the neurectomized adult cat: cellular and behavioral evidence. Neurosci 164:1444-1456.
Dutheil S, Lacour M, Tighilet B (2011) Neurogenic potential of the vestibular nuclei and behavioural recovery time course in the adult cat are governed by the nature of the vestibular damage. PLoS One 6(8):e22262.
Lacour M, Tighilet B (2010) Plastic events in the vestibular nuclei during vestibular compensation: the brain orchestration of a « deafferentation » code. Restor Neurol Neurosci 28(1):19-35. doi:10.3233/RNN-2010-0509 Lacour M, Helmchen C, Vidal PP (2016) Vestibular compensation: the neuro-otologist's best friend. J Neurol 263(1):54-64.
Ris L, de Waele C, Serafin M, Vidal PP, Godaux E (1995) Neuronal activity in the ipsilateral vestibular nucleus following unilateral labyrinthectomy in the alert guinea pig. J Neurophysiol 74(5):2087-2099. doi:10.1152/jn.1995.74.5.2087
Smith PF, Curthoys IS. Mechanisms of recovery following unilateral labyrinthectomy: a review. Brain Res Brain Res Rev (1989) 14(2):155-80.
Tighilet B, Leonard J, Mourre C, Chabbert C (2019) Apamin treatment accelerates equilibrium recovery and gaze stabilization in unilateral vestibular neurectomized cats: Cellular and behavioral aspects. Neuropharmacol 144:133-142.
Tighilet B, Brezun JM, Dit Duflo Sylvie G, Gaubert C, Lacour M (2007) New neurons in the vestibular nuclei complex after unilateral vestibular neurectomy in the adult cat: Reactive neurogenesis in adult vestibular lesioned cats. Eur J Neurosci 25(1):47-58. doi:10.1111/j.1460-9568.2006.05267.x
Tighilet B, Chabbert C (2019) Adult neurogenesis promotes balance recovery after vestibular loss. Progress in Neurobiol Jan 15. pii: S0301-0082(18)30143-6. doi: 10.1016/j.pneurobio.2019.01.001.

## Claims

1. A thyroid hormone for use in the treatment of a peripheral vestibulopathy in a patient in need thereof, the patient not being diagnosed with hypothyroidism.

2. The thyroid hormone for use according to claim 1, wherein the patient is not being diagnosed with hyperthyroidism.

3. The thyroid hormone for use according to any one of claims 1 and 2, wherein the vestibulopathy is a lesional peripheral vestibulopathy.

4. The thyroid hormone for use according to any one of claims 1 to 3, wherein the vestibulopathy is selected from the group consisting of peripheral vestibular neuritis, labyrinthitis or Ménière's disease.

5. The thyroid hormone for use according to any one of claims 1 to 4, wherein the thyroid hormone is triiodothyronine or thyroxine.

6. The thyroid hormone for use according to claim 5, wherein the thyroid hormone is thyroxine.

7. The thyroid hormone for use according to any one of claims 1 to 6, wherein the thyroid hormone is administered in an amount superior or equal to 5 µg/kg, advantageously in an amount ranging from 5 µg/kg to 100 µg/kg.

8. The thyroid hormone for use according to claim 7, wherein the thyroid hormone is administered at a level of approximately 10 µg/kg.

9. The thyroid hormone for use according to any one of claims 1 to 8, wherein the thyroid hormone is administered once a day during the five days following the insult, preferentially during the three days following the insult.

10. A pharmaceutical composition comprising at least a thyroid hormone according to claims 1 to 9, and at least one pharmaceutically acceptable excipient, for use in the treatment of a peripheral vestibulopathy in a patient in need thereof.

11. The pharmaceutical composition for use according to claim 10, wherein the composition is administered by intraperitoneal route.
